(19) Europäisches Patentamt
European Patent Office
Office européen des brevets

(11) Veröffentlichungsnummer : **0 376 083 B1**

(12) # EUROPÄISCHE PATENTSCHRIFT

(45) Veröffentlichungstag der Patentschrift : **03.03.93 Patentblatt 93/09**

(51) Int. Cl.$^5$ : **A61K 7/08,** A61K 7/50, A61K 7/00, C11D 17/00, A61K 7/075

(21) Anmeldenummer : **89123177.1**

(22) Anmeldetag : **14.12.89**

(54) **Fliessfähiges Perlglanzkonzentrat.**

Verbunden mit 90900789.0/0449904 (europäische Anmeldenummer/Veröffentlichungsnummer) durch Entscheidung vom 17.12.91.

(30) Priorität : **23.12.88 DE 3843572**

(43) Veröffentlichungstag der Anmeldung : **04.07.90 Patentblatt 90/27**

(45) Bekanntmachung des Hinweises auf die Patenterteilung : **03.03.93 Patentblatt 93/09**

(84) Benannte Vertragsstaaten : **AT BE CH DE ES FR GB GR IT LI NL SE**

(56) Entgegenhaltungen :
**EP-A- 0 221 465**
**DE-A- 3 519 080**
**DE-A- 3 617 306**

(73) Patentinhaber : **Henkel Kommanditgesellschaft auf Aktien**
**Postfach 1100 Henkelstrasse 67**
**W-4000 Düsseldorf 1 (DE)**

(72) Erfinder : **Kawa, Rolf**
**Humboldtstrasse 47**
**W-4019 Monheim 2 (DE)**
Erfinder : **Ansmann, Achim, Dr.**
**Fichtestrasse 19**
**W-4010 Hilden (DE)**
Erfinder : **Jung, Angelika**
**Poststrasse 24**
**W-4010 Hilden (DE)**

EP 0 376 083 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Perlglanzkonzentrat in Form einer fließ- oder pumpfähigen, wäßrigen Dispersion mit einem Gehalt von 15-40 Gew.-% an perlglanzbildenden Komponenten.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z. B. die Mono- und Diester von Ethylenglykol, Propylenglykol und oligomeren Alkylenglykolen dieser Art oder Glycerin mit $C_{16}$-$C_{22}$-Fettsäuren, Fettsäuren sowie Monoalkanolamide von Fettsäuren mit Alkanolaminen mit 2 oder 3 C-Atomen.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Perlglanzkonzentrate auf Basis der genannten Perlglanzbildner sind z. B. aus DE-A-16 69 152, aus JP-56/71021 (Chem. Abstr. 95/156360), aus DE-A-34 11 328 und DE-A-35 19 081 bekannt.

Die aus DE-A-16 69 152 bekannten Perlglanzkonzentrate enthalten zur Stabilisierung der Dispersion im flüssigen Zustand anionische Tenside. Ein Gehalt an ionogenen Tensiden ist aber bei vielen Anwendungen solcher Perlglanzkonzentrate unerwünscht, da sich mit Rezepturbestandteilen entgegengesetzter Ionogenität Unverträglichkeiten und damit Störungen der Dispersionsstabilität ergeben können.

Weiterhin enthalten die aus diesen Druckschriften bekannten Konzentrate als Teil der perlglanzbildenden Stoffe Fettsäure-monooder -dialkanolamide. Alkanolamine und deren Derivate werden aber in jüngster Zeit verdächtigt, an der Bildung von Nitrosaminen beteiligt zu sein, und es kann daher erwünscht sein, kosmetische Zubereitungen ohne solche Alkanolamine und Alkanolamin-Derivate zu formulieren.

Ein Weglassen der Fettsäurealkanolamide aus den bekannten Perlglanz-Konzentraten führt aber zu einer deutlichen Verringerung der perlglänzenden Eigenschaften. Es wurde daher in der deutschen Patentanmeldung 37 24 547.3 der Anmelderin vorgeschlagen, Perlglanzkonzentrate einzusetzen, die als perlglanzbildenden Stoff im wesentlichen lineare, gesättigte Fettsäuren enthalten. Um einen befriedigenden Perlglanz im Endprodukt zu erhalten, sind jedoch deutlich höhere Konzentrationen an perlglanzbildenden Stoffen notwendig.

Die aus JP-56/71021 bekannten Perlglanzkonzentrate haben den Nachteil, daß sie nicht fließfähig sind und bei entsprechender Verdünnung mit Wasser keine stabilen fließfähigen Dispersionen ergeben. Dadurch wird die Handhabung und technische Verarbeitung der Konzentrate erheblich erschwert.

Es besteht jedoch weiterhin Bedarf an Perlglanzkonzentraten mit hohen Konzentrationen an perlglanzbildenden Komponenten bei gleicher Stabilität, die fließ- oder pumpfähig sind und sich gut in die mit Perlglanz auszustattenden Produkte unabhängig von deren Gehalt an kationischen oder anionischen Komponenten einarbeiten lassen. Weiterhin sollten diese Konzentrate gewünschtenfalls auch frei von Alkanolamiden formulierbar sein und bereits bei üblichen Konzentrationen an perlglanzbildenden Komponenten im Endprodukt diesem den gewünschten Perlglanz verleihen.

Es wurde nun gefunden, daß alle genannten Anforderungen erfüllt werden durch ein Perlglanzkonzentrat in Form einer fließfähigen, wäßrigen Dispersion, das gekennzeichnet ist durch einen Gehalt von

(A) 15 - 40 Gew.-% an perlglanzbildenden Komponenten
(B) 5 - 55 Gew.-% an nichtionogenen, ampholytischen und/oder zwitterionischen Emulgatoren und
(C) 0,1 - 5 Gew.-% an niedermolekularen, mehrwertigen Alkoholen.

Besonders vorteilhafte Eigenschaften zeigen Perlglanzkonzentrate mit einem Gehalt von

(A) 20 - 30 Gew.-% an perlglanzbildenden Komponenten
(B) 15 - 30 Gew.-% an nichtionogenen, ampholytischen und/oder zwitterionischen Emulgatoren und
(C) 0,5 -3 Gew.-% an niedermolekularen, mehrwertigen Alkoholen.

Unter perlglanzbildenden Komponenten werden schmelzbare Fett- oder Wachskörper verstanden, die beim Abkühlen ihrer wäßrigen Lösungen oder Emulsionen in einem Temperaturbereich von etwa 30 - 90 °C in Form feiner, perlglänzender Körper auskristallisieren.

Bevorzugte perlglanzbildende Komponenten sind

(A1) Ester der Formel (I),

$$R^1 - (OC_nH_{2n})_x - OR^2 \qquad (I)$$

in der $R^1$ eine lineare Fettacylgruppe mit 14 bis 22 C-Atomen, $R^2$ Wasserstoff oder eine Gruppe $R^1$, n = 2 oder 3 und x eine Zahl von 1 bis 4 ist,

(A2) Monoalkanolamide der allgemeinen Formel (II),

$$R^3 - CO - NH - X \qquad (II)$$

in der $R^3$ eine Alkylgruppe mit 8 bis 22 C-Atomen, insbesondere mit 8 bis 18 C-Atomen, und X eine Gruppe

$-CH_2-CH_2-OH$, eine Gruppe $-CH_2-CH_2-CH_2-OH$ oder eine Gruppe $-C(CH_3)_2-OH$ darstellt,

(A3) lineare, gesättigte Fettsäuren mit 16 bis 22 C-Atomen

und

(A4) β-Ketosulfone der allgemeinen Formel (III),

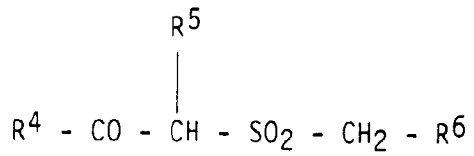

(III)

in der $R^4$ eine Alkyl- oder Alkenylgruppe mit 11 bis 21 C-Atomen, $R^5$ und $R^6$ Wasserstoffatome oder gemeinsam eine Ethylengruppe, die mit der zwischen $R^5$ und $R^6$ liegenden Gruppe einen Tetrahydrothiophendioxidring bildet, darstellen.

Die erfindungsgemäßen Perlglanzkonzentrate können sowohl ausschließlich Vertreter einer dieser Verbindungsklassen als auch Mischungen von Vertretern mehrerer dieser Verbindungsklassen enthalten.

Als Ester (A1) der allgemeinen Formel $R^1(OC_nH_{2n})_xOR^2$ können z. B. die Mono- und Diester des Ethylenglykols und Propylenglykols mit höheren Fettsäuren, z. B. mit Palmitinsäure, Stearinsäure oder Behensäure oder die Diester des Diethylenglykols oder des Triethylenglykols mit solchen Fettsäuren eingesetzt werden. Geeignet sind auch Mischungen von Mono- und Diestern der genannten Glykole mit Fettsäuregemischen, z. B. mit gehärteter Talgfettsäure oder mit der gesättigten $C_{14}$-$C_{18}$-Fettsäurefraktion der Talgfettsäure. Bevorzugt geeignet sind der Ethylenglykolmono- und/oder -diester der Palmitin- und/oder Stearinsäure.

Bevorzugte Monoalkanolamide (A2) sind die Monoethanolamide. Diese Verbindungen können einen einheitlichen Alkylrest enthalten. Es ist jedoch üblich, bei der Herstellung der Alkanolamide von Fettsäuregemischen aus natürlichen Quellen, z.B. Kokosfettsäuren, auszugehen, so daß entsprechende Mischungen bezüglich der Alkylreste vorliegen.

Als lineare Fettsäuren (A3) können z. B. Palmitinsäure, Stearinsäure, Arachinsäure oder Behensäure eingesetzt werden, geeignet sind aber auch technische Fettsäureschnitte, die ganz oder überwiegend aus Fettsäuren mit 16 bis 22 C-Atomen bestehen, z. B. Palmitin-Stearinsäure-Fraktionen wie sie aus Talgfettsäure durch Abtrennung der bei +5 °C flüssigen Fettsäuren gewonnen werden oder Palmitin-Stearinsäure-Fraktionen wie sie durch Härten von Talgfettsäure erhältlich sind.

Gegenüber den bekannten Ethylenglykolmono- und -diestern haben die β-Ketosulfone (A4) der allgemeinen Formel (III) den Vorteil, daß der Perlglanz der Zubereitungen eine höhere Thermostabilität aufweist, d.h. daß der Perlglanz beim Erwärmen der Zubereitungen auf über 50 °C, teilweise sogar auf über 70 °C, über mehrere Stunden erhalten bleibt. Hinsichtlich weiterer Informationen zu den genannten β-Ketosulfonen wird ausdrücklich auf den Inhalt der deutschen Patentanmeldung 35 08 051 hingewiesen.

Im Rahmen der erfindungsgemäßen Lehre ist es bevorzugt, die stark perlglanzgebenden Verbindungen der Klassen (A1) und (A2) zu verwenden.

Ganz besonders bevorzugt sind solche Perlglanzkonzentrate, deren perlglanzbildende Komponenten zu mindestens 70 Gew.-%, insbesondere zu mindestens 90 Gew.-%, aus Ethylenglykoldistearat bestehen.

Als Emulgatoren (B) können nichtionogene, ampholytische und/oder zwitterionische grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein.

Nichtionogene Emulgatoren enthalten als hydrophile Gruppe z. B. eine Polyolgruppe, eine Polyalkylenglykolethergruppe oder eine Kombination aus Polyol- und Polyglykolethergruppe.

Bevorzugt sind solche Perlglanzkonzentrate, die als Emulgatoren nichtionogene Tenside aus der Gruppe der

(B1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

(B2) $C_{12}$-$C_{18}$-Fettsäuremono- und -diester von Anlagerungsprodukten von 1 bis 30 Mol Ethylenoxid an Glycerin,

(B3) Glycerinmono- und -diester und Sorbitanmono- und -diester von gesättigten und ungesättigten $C_8$-$C_{18}$-Fettsäuren und deren Ethylenoxidanlagerungsprodukte,

(B4) $C_8$-$C_{18}$-Alkylmono- und -oligoglycoside und deren ethoxylierte Analoga

und

(B5) Anlagerungsprodukte von 10 bis 60 Mol Ethylenoxid an Ricinus-öl und gehärtetes Ricinusöl, enthalten.

Ebenfalls geeignet sind Mischungen von Verbindungen aus mehreren dieser Substanzklassen.

Die Anlagerungsprodukte von Ethylenoxid und/oder von Propylenoxid an Fettalkohole, Fettsäuren, Alkylphenole, Glycerin-mono- und -diester sowie Sorbitanmono- und -diester von Fettsäuren oder an Ricinusöl stellen bekannte, im Handel erhätliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

$C_{12}$-$C_{18}$-Fettsäuremono- und -diester von Anlagerungsprodukten von Ethylenoxid an Glycerin sind aus DE-PS 20 24 051 als Rückfettungsmittel für kosmetische Zubereitungen bekannt. $C_8$-$C_{18}$-Alkylmonound oligoglycoside, ihre Herstellung und ihre Verwendung als oberflächenaktive Stoffe sind beispielsweise aus US-A 3,839,318, US-A 3,707,535, US-A 3,547,828, DE-A 19 43 689, DE-A 20 36 472 und DE-A 30 01 064 sowie EP-A 77 167 bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Die Verbindungen aus der Gruppe (B1) stellen besonders bevorzugte nichtionogene Emulgatoren (B) im Rahmen der erfindungsgemäßen Lehre dar.

Weiterhin können als Emulgatoren (B) zwitterionische Tenside verwendet werden. Als zwitterionische Tenside werden solche oberflächenaktiven Verbindungen bezeichnet, die im Molekül mindestens eine quartäre Ammoniumgruppe und mindestens eine -$COO^{(-)}$- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat.

Ebenfalls geeignete Emulgatoren (B) sind ampholytische Tenside. Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Im Rahmen der erfindungsgemäßen Lehre können die Perlglanzkonzentrate Vertreter einer oder mehrerer der genannten Tensidklassen enthalten. Bei Verwendung von Mischungen ist es bevorzugt, nichtionogene und zwitterionische und/oder ampholytische Tenside in einem Massenverhältnis von 5 : 1 bis 1 : 5 einzusetzen.

Die erfindungsgemäßen Perlglanzkonzentrate, die lediglich nichtionogene, zwitterionische und/oder ampholytische Tenside enthalten, haben sich als besonders universell einsetzbar und mit wäßrigen Zubereitungen wasserlöslicher oberflächenaktiver Stoffe beliebiger Art und Ionogenität als besonders gut verträglich erwiesen.

Gewünschtenfalls können die Perlglanzkonzentrate aber zusätzlich noch anionische oder kationische Emulgatoren enthalten.

Solche anionischen Emulgatoren sind beispielsweise Alkylsulfate und Alkylpolyethylenglykolethersulfate mit 1 bis 6 Ethylenglykolethergruppen im Molekül, die in Form ihrer Alkali-, Magnesium-, Ammonium-, Mono-, Di- oder Trialkanolammoniumsalze mit 2 bis 3 C-Atomen in der Alkanolgruppe eingesetzt werden. Weitere geeignete Aniontenside sind Alkansulfonate, $\alpha$-Olefinsulfonate, $\alpha$-Sulfofettsäuremethylester, Fettalkohol(polyglykolether)carboxylate, Sulfobernsteinsäuremono- und dialkylester, Sulfobernsteinsäureester-Salze, Acylisethionate, Acyltauride und Acylsarcoside. Auch Seifen können als Emulgatoren verwendet werden. Dies kann z. B. dadurch erreicht werden, daß man einen kleinen Teil, d.h. etwa 1 bis 20 Gew.-%, der linearen, gesättigten Fettsäuren durch zugesetztes Alkalihydroxid verseift und auf diese Weise in einen anionischen Emulgator überführt. Bevorzugte anionische Tenside sind die Alkylpolyethylenglykolethersulfate wie beispielsweise das Natriumlaurylpolyglykolethersulfat.

Als kationische Emulgatoren sind quartäre Ammoniumtenside, beispielsweise Alkyltrimethylammoniumchloride und Dialkyldimethylammoniumchloride, z. B. Cetyltrimethylammoniumchlorid, Stearyltrimethylammoniumchlorid, Distearyldimethylammoniumchlorid, Lauryldimethylammoniumchlorid und Lauryldimethylbenzylammoniumchlorid, Cetylpyridiniumchlorid und Talgalkyltris-(oligooxyalkyl)-ammonium-phosphat zu nennen.

Die Alkylgruppen in den genannten anionischen und kationischen Tensiden enthalten üblicherweise 8 bis 22, insbesondere 12 bis 18 C-Atome.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Entscheidend für die Fließ- bzw. Pumpfähigkeit der erfindungsgemäßen Perlglanzkonzentrate ist der Gehalt an niedermolekularen, mehrwertigen Alkoholen. Bevorzugt eingesetzte niedermolekulare mehrwertige Alkohole enthalten 2-6 Kohlenstoffatome und 2-6 Hydroxylgruppen. Solche Alkohole sind beispielsweise Ethylenglykol, 1,2- und 1,3-Propylenglykol, Glycerin, Di- und Triethylenglykol, Erythrit, Arabit, Adonit, Xylit, Sorbit, Mannit und Dulcit. Die Verwendung von bei Raumtemperatur flüssigen Verbindungen, insbesondere von 1,2-Propylenglykol und/oder Glycerin, ist besonders bevorzugt.

Bei Perlglanzkonzentraten, die weniger als etwa 30 Gew.-% an perlglanzgebenden Komponenten enthalten, hat es sich in vielen Fällen als ausreichend erwiesen, wenn der Gehalt an niedermolekularen, mehrwertigen Alkoholen etwa 1 Gew.-% beträgt. Dies trifft vor allem dann zu, wenn als Alkoholkomponente 1,2-Propylenglykol und/oder Glycerin verwendet wird.

Daneben enthalten die erfindungsgemäßen Perlglanzkonzentrate im wesentlichen Wasser. Zur Konservierung gegen Bakterien- und Pilzbefall werden handelsübliche Konservierungsmittel in untergeordneten Mengen zugegeben. Weiterhin können in untergeordneten Mengen Puffersubstanzen zur Einstellung des pH-Wertes auf Werte zwischen 2 und 8, z. B. Citronensäure und/oder Natriumcitrat enthalten sein.

Die erfindungsgemäßen Perlglanzkonzentrate sind mindestens in einem Temperaturbereich von 5 - 40 °C pumpbar und über einen längeren Zeitraum , d. h. mindestens etwa 3 Monate lagerstabil.

Die Herstellung der erfindungsgemäßen Perlglanzkonzentrate erfolgt bevorzugt in der Weise, daß die Komponenten (A), (B) und (C) zunächst gemeinsam auf eine Temperatur erwärmt wird, die etwa 1 bis 30 °C oberhalb des Schmelzpunktes liegt. Dies wird in den meisten Fällen eine Temperatur von etwa 60 bis 90 °C sein. Sodann wird zu dieser Mischung das auf etwa die gleiche Temperatur erwärmte Wasser hinzugegeben. Falls als Emulgator ein ionisches, wasserlösliches Tensid eingesetzt wird, kann es bevorzugt sein, dieses in der Wasserphase aufzulösen und mit dem Wasser zusammen in die Mischung einzubringen. Die wäßrige Phase kann auch bereits gegebenenfalls die Puffersubstanzen gelöst enthalten. Die entstehende Dispersion wird dann unter stetigem Rühren auf Raumtemperatur, d. h. auf etwa 25 °C, abgekühlt. Die Viskosität des Perlglanzkonzentrates ist in den allermeisten Fällen so niedrig, daß auf den Einsatz besonderer Rühraggregate wie Homogenisatoren oder andere hochtourige Mischvorrichtungen verzichtet werden kann. Temperaturempfindliche Konservierungsmittel sollten erst nach Abkühlung auf Temperaturen unterhalb 40 °C, insbesondere erst kurz vor Beendigung des Abkühlvorganges bei Temperaturen von etwa 30 °C zugesetzt werden.

Die erfindungsgemäßen Perlglanzkonzentrate eignen sich zur Herstellung getrübter und perlglänzender flüssiger, wäßriger Zubereitungen wasserlöslicher oberflächenaktiver Stoffe. Sie können z.B. in flüssige Wasch- und Reinigungsmittel wie Spülmittel, flüssige Feinwaschmittel und flüssige Seifen, bevorzugt aber in flüssige Körperreinigungs- und Pflegemittel wie z.B. Haarwaschmittel (Shampoos), flüssige Hand- und Körperwaschmittel, Duschbadzubereitungen, Badezusätze (Schaumbäder), Haarspülmittel oder Haarfärbezubereitungen eingearbeitet werden.

Zur Erzeugung von Perlglanz werden den klaren wäßrigen Zübereitungen bei 0 bis 40 °C die erfindungsgemäßen Perlglanzkonzentrate in einer Menge von 1 bis 10 Gew.-%, insbesondere 1,5 bis 5 Gew.-%, der Zubereitung zugesetzt und unter Rühren darin verteilt. Je nach Zubereitung und Einsatzkonzentration entsteht ein metallisch glänzender, dichter bis schwach glänzender, extrem dichter Perlglanz.

Die folgenden Beispiele sollen den Erfindungsgegenstand näher erläutern, ohne ihn darauf zu begrenzen.

<u>Beispiele</u>

Es wurden fließfähige Perlglanzkonzentrate mit den in Tabelle 1 aufgeführten Zusammensetzungen hergestellt. Soweit verdünnte Lösungen der Komponenten (A) und (B) eingesetzt wurden, beziehen sich die Angaben auf Gew.-% Aktivsubstanz. Bei den mit den Verkaufsbezeichnungen aufgeführten Stoffen handelt es sich um die im folgenden aufgeführten Substanzen:

[1] Ethylenglykoldistearat (mind. 90 % Diester) (HENKEL)

[2] Kokosfettsäuremonoethanolamid (ca. 95 % Amid) (HENKEL)
Zusammensetzung der Fettsäure:
- ca. 56 % Laurinsäure
- ca. 21 % Myristinsäure
- ca. 10 % Palmitinsäure
- ca. 13 % Stearinsäure und Ölsäure

[3] $C_{12}$-$C_{14}$-Fettalkohol + 4 Ethylenoxid (HENKEL)
[4] Wäßrige Lösung eines Fettsäureamid-Derivats mit Betainstruktur der Formel $R-CONH-(CH_2)_3-N^+(CH_3)_2-CH_2-COO^-$ mit der CTFA-Bezeichnung Cocamidopropyl Betaine (ca. 30 % Aktivsubstanz, ca. 5 % NaCl) (HENKEL)
[5] $C_{12}$-$C_{14}$-Fettalkohol + 3 Ethylenoxid (HENKEL)
[6] 86 % in Wasser

Tabelle 1: Fließfähige Perlglanzkonzentrate

Gehalt [Gew.-%]

| Komponenten | Mischung Nr. 1 | 2 | 3 | 4 | 5 | 6 | 7 | 8 | 9 | 10 | 11 | 12 |
|---|---|---|---|---|---|---|---|---|---|---|---|---|
| Perlglanzbildner (A) | 25 | 25 | 25 | 30 | 30 | 30 | 25 | 25 | 25 | 25 | 25 | 25 |
| davon: | | | | | | | | | | | | |
| Cutina(R) AGS[1] | 25 | 25 | 25 | 30 | 30 | 25 | 25 | 25 | 25 | 25 | 25 | 25 |
| Comperlan(R) 100[2] | - | - | - | - | - | 5 | - | - | - | - | - | - |
| Emulgator (B) | 19 | 17 | 19 | 20,5 | 20,5 | 20,5 | 17 | 18 | 17 | 17 | 19 | 18,5 |
| davon: | | | | | | | | | | | | |
| Dehydol(R) LS4[3] | 5 | 11 | 5 | 13 | 13 | 13 | 11 | 8 | 11 | 11 | 5 | 11 |
| Dehyton(R) K[4] | 9 | 6 | 9 | 7,5 | 7,5 | 7,5 | 6 | 6 | 6 | 6 | 9 | 7,5 |
| Dehydol(R) LS3[5] | 5 | - | 5 | - | - | - | - | 4 | - | - | 5 | - |
| Alkohol (C) | 1 | 5 | 1 | 5 | 5 | 5 | 5 | 1 | 1 | 1 | 1 | 5 |
| davon: | | | | | | | | | | | | |
| Glycerin[6] | - | 5 | 1 | 5 | - | 5 | - | - | 1 | - | 1 | 3 |
| 1,2-Propylenglykol | 1 | - | - | - | 5 | - | 5 | 1 | - | 1 | - | 2 |
| Wasser<br>Konservierungsmittel<br>Stellmittel | <——— ad 100 ———> | | | | | | | | | | | |

Die eingesetzten Mengen an Komponenten A, B und C wurden auf eine Temperatur von 75 °C erwärmt. Zu dieser Schmelze wurde das auf 75 °C erwärmte Wasser hinzugegeben. Unter stetigem Rühren wurde die Dispersion dann auf 25 °C abgekühlt, wobei bei einer Temperatur von 30 °C das Konservierungsmittel zugegeben wurde.

Die Viskositäten der Perlglanzkonzentrate wurden mit einem Brookfield RVF-Viskosimeter, Spindel 5, 10 Umdrehungen pro Minute, jeweils bei der Temperatur bestimmt, bei der die Mischung gelagert worden war. Die Vergleichsmischung Vx hat jeweils bis auf die fehlende Komponente C die gleiche Zusammensetzung wie die Mischung x. Die Meßwerte sind in Tabelle 2 zusammengestellt.

Tabelle 2: Viskositätswerte

| Mischung Nr. | Temperatur [°C] | Lagerzeit [Tage] | Viskosität [mPas] |
|---|---|---|---|
| 2 | 10 | 1 | 14400 |
| 2 | 25 | 1 | 6000 |
| 2 | 40 | 1 | 14000 |
| 7 | 10 | 1 | 14000 |
| 7 | 25 | 1 | 5600 |
| 7 | 40 | 1 | 8000 |
| 9 | 10 | 14 | 14000 |
| V9 | 10 | 14 | 22000 |
| 9 | 25 | 1 | 6000 |
| V9 | 25 | 1 | 11200 |
| 9 | 25 | 7 | 5200 |
| V9 | 25 | 7 | 12000 |
| 9 | 25 | 14 | 5200 |
| V9 | 25 | 14 | 12000 |
| 9 | 40 | 14 | 14000 |
| V9 | 40 | 14 | 20000 |
| 10 | 10 | 14 | 16000 |
| V10 | 10 | 14 | 22000 |
| 10 | 25 | 1 | 6000 |
| V10 | 25 | 1 | 11200 |
| 10 | 25 | 7 | 6000 |
| V10 | 25 | 7 | 12000 |
| 10 | 25 | 14 | 6400 |
| V10 | 25 | 14 | 12000 |
| 10 | 40 | 14 | 13600 |
| V10 | 40 | 14 | 20000 |

Die Ergebnisse zeigen die deutliche Senkung der Viskosität durch den Alkoholzusatz.

Anwendungsbeispiele:

1) Shampoo mit Aniontensiden

| Komponente | | Gew.-% |
|---|---|---|
| Fettalkohol($C_{12}$-$C_{14}$)-polyglykol(2 EO)-ethersulfat, Natriumsalz, ca. 30 % in Wasser (CTFA-Bezeichnung: Sodium Laureth Sulfate | | 40,0 |
| N-Kokosacylamidopropyl-dimethylglycin, 30 % in Wasser (CTFA-Bezeichnung: Cocamidopropyl Betain) | | 10,0 |
| Cetiol(R) HE[7] | | 2,0 |
| Perlglanzkonzentrat Mischung 9 | | 3,0 |
| Natriumchlorid | | 0,8 |
| Wasser | ad | 100 |

2) Schaumbat mit Aniontensiden

| Komponente | | Gew.-% |
|---|---|---|
| Fettalkohol($C_{12}$-$C_{14}$)-sulfat, Magnesiumsalz, ca. 30 % in Wasser (CTFA-Bezeichnung: Magnesium Lauryl Sulfate) | | 40,0 |
| N-Kokosacylamidopropyl-dimethylglycin, 30 % in Wasser (CTFA-Bezeichnung: Cocamidopropyl Betain) | | 10,0 |
| Sulfobernsteinsäuremonolaurylpolyglykol(3 EO)-ester, 40 % in Wasser (CTFA-Bezeichnung: Disodium Laureth-sulfosuccinate) | | 4,5 |
| Cetiol(R) HE[7] | | 2,0 |
| Perlglanzkonzentrat Mischung 7 | | 3,0 |
| Natriumchlorid | | 0,3 |
| Wasser | ad | 100 |

3) Haarkur mit kationischen Tensiden

| Komponente | | Gew.-% |
|---|---|---|
| Quaternum-52[8] | | 2,0 |
| Cetiol(R) HE | | 0,5 |
| Viscontran(R) HEC 30000 PR[9] | | 50,0 |
| Perlglanzkonzentrat Mischung 2 | | 5,0 |
| Citronensäure | | 0,2 |
| Wasser | ad | 100 |

[7] Polyol-Fettsäureester (CTFA-Bezeichnung: PEG-7-Glyceryl Cocoate) (HENKEL)

[8] Tris-(oligooxyethyl)-alkylammoniumphosphat, 50 % in Wasser (HENKEL)

[9] Hydroxyethylcellulose, 2 % in Wasser (AQUALON)

**Patentansprüche**

1. Perlglanzkonzentrat in Form einer fließfähigen, wäßrigen Dispersion, dadurch gekennzeichnet, daß es
   (A) 15 - 40 Gew.-% an perlglanzbildenden Komponenten
   (B) 5 - 55 Gew.-% an nichtionogenen, ampholytischen und/oder zwitterionischen Emulgatoren und
   (C) 0,1 -5 Gew.-% an niedermolekularen, mehrwertigen Alkoholen
   enthält.

2. Perlglanzkonzentrat nach Anspruch 1, dadurch gekennzeichnet, daß es
   (A) 20 - 30 Gew.-% an perlglanzbildenden Komponenten
   (B) 15 - 30 Gew.-% an nichtionogenen, ampholytischen und/oder zwitterionischen Emulgatoren und
   (C) 0,5 -3 Gew.-% an niedermolekularen, mehrwertigen Alkoholen
   enthält.

3. Perlglanzkonzentrat nach einem der Ansprüche 1 oder 2, dadurch gekennzeichnet, daß als perlglanzbildende Komponenten
   (A1) Ester der Formel (I),
   $$R^1 - (OC_nH_{2n})_x - OR^2 \qquad (I)$$
   in der $R^1$ eine lineare Fettacylgruppe mit 14 bis 22 C-Atomen, $R^2$ Wasserstoff oder eine Gruppe $R^1$, n = 2 oder 3 und x eine Zahl von 1 bis 4 ist,
   und/oder
   (A2) Monoalkanolamide der allgemeinen Formel (II),
   $$R^3 - CO - NH - X \qquad (II)$$
   in der $R^3$ eine Alkylgruppe mit 8 bis 22 C-Atomen, insbesondere mit 8 bis 18 C-Atomen, und X eine Gruppe $-CH_2-CH_2-OH$, eine Gruppe $-CH_2-CH_2-CH_2-OH$ oder eine Gruppe $-C(CH_3)_2-OH$ darstellt,
   (A3) lineare, gesättigte Fettsäuren mit 16 bis 22 C-Atomen
   und/oder

(A4) β-Ketosulfone der allgemeinen Formel (III),

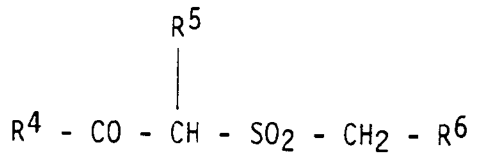

(III)

in der $R^4$ eine Alkyl- oder Alkenylgruppe mit 11 bis 21 C-Atomen, $R^5$ und $R^6$ Wasserstoffatome oder gemeinsam eine Ethylengruppe, die mit der zwischen $R^5$ und $R^6$ liegenden Gruppe einen Tetrahydrothiophendioxidring bildet, darstellen,

enthalten sind.

4. Perlglanzkonzentrat nach Anspruch 3, dadurch gekennzeichnet, daß die perlglanzbildenden Komponenten zu mindestens 70 Gew.-%, insbesondere zu mindestens 90 Gew.-%, aus Ethylenglykoldistearat bestehen.

5. Perlglanzkonzentrat nach einem der Ansprüche 1 bis 4, dadurch gekennzeichnet, daß als Emulgatoren nichtionogene Tenside, insbesondere aus der Gruppe der

(B1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

(B2) $C_{12}$-$C_{18}$-Fettsäuremono- und -diester von Anlagerungsprodukten von 1 bis 30 Mol Ethylenoxid an Glycerin,

(B3) Glycerinmono- und -diester und Sorbitanmono- und -diester von gesättigten und ungesättigten $C_8$-$C_{18}$-Fettsäuren und deren Ethylenoxidanlagerungsprodukte,

(B4) $C_8$-$C_{18}$-Alkylmono- und -oligoglycoside und deren etholxylierte Analoga

und

(B5) Anlagerungsprodukte von 10 bis 60 Mol Ethylenoxid an Ricinusöl und gehärtetes Ricinusöl,

enthalten sind.

6. Perlglanzkonzentrat nach einem der Ansprüche 1 bis 5, dadurch gekennzeichnet, daß als Emulgatoren zwitterionische Tenside, insbesondere ausgewählt aus der Gruppe der Betaine, enthalten sind.

7. Perlglanzkonzentrat nach einem der Ansprüche 1 bis 6, dadurch gekennzeichnet, daß als Emulgatoren ampholytische Tenside, enthalten sind.

8. Perlglanzkonzentrat nach einem der Ansprüche 1 bis 7, dadurch gekennzeichnet, daß nichtionogene und zwitterionische und/oder ampholytische Tenside in einem Massenverhältnis von 5 : 1 bis 1 : 5 enthalten sind.

9. Perlglanzkonzentrat nach einem der Ansprüche 1 bis 8, dadurch gekennzeichnet, daß der niedermolekulare, mehrwertige Alkohol 2 - 6 C-Atome und 2 - 6 Hydroxylgruppen enthält.

10. Perlglanzkonzentrat nach Anspruch 9, dadurch gekennzeichnet, daß als niedermolekularer, mehrwertiger Alkohol 1,2-Propylenglykol und/oder Glycerin enthalten ist.

11. Verfahren zur Herstellung von Perlglanzkonzentraten nach einem der Ansprüche 1 bis 10, dadurch gekennzeichnet, daß man eine Mischung der Komponenten (A), (B) und (C) auf eine Temperatur erwärmt, die 1 bis 30 °C oberhalb des Schmelzpunktes der Mischung liegt, mit der notwendigen Menge Wasser etwa der gleichen Temperatur mischt und anschließend auf Raumtemperatur abkühlt.

12. Verfahren zur Herstellung getrübter und perlglänzender flüssiger, wäßriger Zubereitungen wasserlöslicher oberflächenaktiver Stoffe, dadurch gekennzeichnet, daß man den klaren wäßrigen Zubereitungen bei 0 bis 40 °C Perlglanzkonzentrate nach einem der Ansprüche 1 bis 10 in einer Menge von 0,5 bis 10 Gew.-%, insbesondere von 1,5 bis 5 Gew.-%, der Zubereitung zusetzt und unter Rühren darin verteilt.

## Claims

1. A pearlescent concentrate in the form of a free-flowing, aqueous dispersion, characterized in that it contains
   (A) 15 to 40% by weight pearlescing components,
   (B) 5 to 55% hy weight nonionic, ampholytic and/or zwitterionic emulsifiers and
   (C) 0.1 to 5% by weight low molecular weight, polyhydric alcohols.

2. A pearlescent concentrate as claimed in claim 1, characterized in that it contains
   (A) 20 to 30% by weight pearlescing components,
   (B) 15 to 30% hy weight nonionic, ampholytic and/or zwitterionic emulsifiers and
   (C) 0.5 to 3% hy weight low molecular weight, polyhydric alcohols.

3. A pearlescent concentrate as claimed in claim 1 or 2, characterized in that it contains as pearlescing components
   (A1) esters corresponding to formula (I)
   $$R^1 - (OC_nH_{2n})_x - OR^2 \qquad (I)$$
   in which $R^1$ is a linear $C_{14-22}$ fatty acyl group, $R^2$ is hydrogen or a group $R^1$, n = 2 or 3 and x is a number of from 1 to 4,
   (A2) monoalkanolamides corresponding to general formula (II)
   $$R^3 - CO - NH - X \qquad (II)$$
   in which $R^3$ is an alkyl group containing 8 to 22 and, more especially, 8 to 18 carhon atoms and X is a group $-CH_2-CH_2-OH$, a group $-CH_2-CH_2-CH_2-OH$ or a group $-C(CH_3)_2-OH$,
   (A3) linear, saturated $C_{16-22}$ fatty acids and
   (A4) β-ketosulfones corresponding to general formula (III)

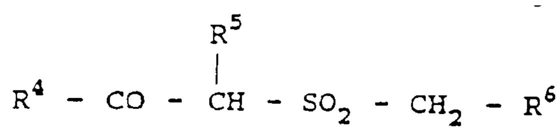

   in which $R^4$ is a $C_{11-21}$ alkyl or alkenyl group, $R^5$ and $R^6$ are hydrogen atoms or together represent an ethylene group which forms a tetrahydrothiophene dioxide ring with the group between $R^5$ and $R^6$.

4. A pearlescent concentrate as claimed in claim 3, characterized in that at least 70% by weight and, in particular, at least 90% by weight of the pearlescing components consist of ethylene glycol distearate.

5. A pearlescent concentrate as claimed in any of claims 1 to 4, characterized in that it contains nonionic surfactants as emulsifiers, more especially nonionic surfactants from the group consisting of
   (B1) adducts of 2 to 30 mol ethylene oxide and/or 0 to 5 mol propylene oxide with linear $C_{8-22}$ fatty alcohols, with $C_{12-22}$ fatty acids and with alkyl phenols containing 8 to 15 carbon atoms in the alkyl group,
   (B2) $C_{12-18}$ fatty acid monoesters and diesters of adducts of 1 to 30 mol ethylene oxide with glycerol,
   (B3) glycerol monoesters and diesters and sorbitan monoesters and diesters of saturated and unsaturated $C_{8-18}$ fatty acids and ethylene oxide adducts thereof,
   (B4) $C_{8-18}$ alkyl mono- and oligo-glycosides and ethoxylated analogs thereof and
   (B5) adducts of 10 to 60 mol ethylene oxide with castor oil and hydrogenated castor oil.

6. A pearlescent concentrate as claimed in any of claims 1 to 5, characterized in that zwitterionic surfactants, more especially selected from the group of betaines, are present as emulsifiers.

7. A pearlescent concentrate as claimed in any of claims 1 to 6, characterized in that ampholytic surfactants are present as emulsifiers.

8. A pearlescent concentrate as claimed in any of claims 1 to 7, characterized in that nonionic and zwitterionic and/or ampholytic surfactants are present in a ratio by weight of 5:1 to 1:5.

9. A pearlescent concentrate as claimed in any of claims 1 to 8, characterized in that the low molecular weight

polyhydric alcohol contains 2 to 6 carbon atoms and 2 to 6 hydroxyl groups.

**10.** A pearlescent concentrate as claimed in claim 9, characterized in that 1,2-propylene glycol and/or glycerol is present as the low molecular weight polyhydric alcohol.

**11.** A process for the production of the pearlescent concentrates claimed in any of claims 1 to 10, characterized in that a mixture of components (A), (B) and (C) is heated to a temperature 1 to 30°C above the melting point of the mixture, mixed with the necessary quantity of water at substantially the same temperature and the resulting mixture subsequently cooled to room temperature.

**12.** A process for the production of clouded and pearlescent free-flowing, aqueous preparations of water-soluble surfactants, characterized in that the, pearlescent concentrates claimed in any of claims 1 to 10 are added to the clear aqueous preparations at 0 to 40°C in a quantity of from 0.5 to 10% by weight and more especially in a quantity of from 1.5 to 5% by weight and are dispersed therein with stirring.

## Revendications

**1.** Concentré d'agents nacrants, sous la forme d'une dispersion aqueuse coulante, qui se caractérise par une concentration de
(A) 15 à 40 % en poids de composants nacrants
(B) 5 à 55 % en poids d'émulsifiants non ioniques, ampholytes et/ou zwitterioniques et
(C) 0,1 à 5 % en poids d'alcools polyvalents ou polyols de faible poids moléculaire.

**2.** Concentré d'agents nacrants, selon la revendication 1, caractérisé en ce qu'il renferme
(A) 20 à 30 % en poids de composants nacrants
(B) 15 à 30 % en poids d'émulsifiants non ioniques, ampholytes et/ou zwitterioniques et
(C) 0,5 à 3 % en poids d'alcools polyvalents de faible poids moléculaire

**3.** Concentré d'agents nacrants, selon l'une des revendications 1 ou 2, caractérisé en ce sont contenus comme composants nacrants,
(A1) des esters de la formule (I)

$$R^1 - (OC_nH_{2n})_x - OR^2 \qquad (I)$$

dans laquelle $R^1$ représente un groupe acyle gras linéaire comportant 14 à 22 atomes de C, $R^2$ correspond à l'hydrogène ou à un groupe $R^1$, n = 2 ou 3 et x est un nombre de 1 à 4,
et/ou
(A2) des monoalcanolamides de la formule générale (II)

$$R^3 - CO - NH - X \qquad (II)$$

dans laquelle $R^3$ représente un groupe alkyle comportant 8 à 22 atomes de C, en particulier comportant 8 à 18 atomes de C, tandis que X est un groupe $-CH_2-CH_2-OH$, un groupe $-CH_2-CH_2-CH_2-OH$ ou un groupe $-C(CH_3)_2-OH$,
(A3) des acides gras linéaires saturés comportant 16 à 22 atomes de C
et/ou
(A4) des bêta-cétosulfones de la formule générale (III)

$$R^4 - CO - \underset{}{\overset{\displaystyle R^5}{\overset{|}{CH}}} - SO_2 - R^6 \qquad (III)$$

dans laquelle $R^4$ représente un groupe alkyle ou alcényle comportant 11 à 21 atomes de C, $R^5$ et $R^6$ sont des atomes d'hydrogène ou représentent ensemble un groupe éthylène, qui forme un noyau tétrahydrothiophènedioxide avec le groupe situé entre $R^5$ et $R^6$.

**4.** Concentré d'agents nacrants, selon la revendication 3, caractérisé en ce que les composants nacrants sont constitués à au moins 70 % en poids, en particulier à au moins 90 % en poids, de distéarate d'éthylèneglycol.

5. Concentré d'agents nacrants, selon l'une des revendications 1 à 4, caractérisé en ce que sont contenus comme émulsifiants, des tensioactifs non ioniques, en particulier faisant partie du groupe
(B1) des produits d'addition de 2 à 30 moles d'oxyde d'éthylène et/ou de 0 à 5 moles d'oxyde de propylène à des alcools gras linéaires comportant 8 à 22 atomes de C, à des acides gras comprenant 12 à 22 atomes de C et à des alkylphénols dont le groupe alkyle comporte 8 à 15 atomes de C.
(B2) des mono- et des diesters d'acides gras en $C_{12}$ à $C_{18}$ de produits d'addition de 1 à 30 moles d'oxyde d'éthylène à de la glycérine,
(B3) des mono- et des diesters de glycérine et des-mono- et des diesters de sorbitane d'acides gras saturés et insaturés en $C_8$ à $C_{18}$ et leurs produits d'addition d'oxyde d'éthylène,
(B4) des alkyl (en $C_8$ à $C_{18}$)-mono- et oligoglucosides et leurs analogues éthoxylés
et
(B5) des produits d'addition de 13 à 60 moles d'oxyde d'éthylène à de l'huile de ricin et à de l'huile de ricin durcie.

6. Concentré d'agents nacrants, selon l'une des revendications 1 à 5, caractérisé en ce que sont contenus comme émulsifiants, des tensioactifs zwitterioniques, en particulier sélectionnés dans le groupe des bétaïnes.

7. Concentré d'agents nacrants, selon l'une des revendications 1 à 6, caractérisé en ce que sont contenus comme émulsifiants, des tensioactifs ampholytes.

8. Concentré d'agents nacrants, selon l'une des revendications 1 à 7, caractérisé en ce que les tensioactifs non ioniques et zwitterioniques et/ou ampholytes sont contenus dans un rapport massique compris entre 5:1 et 1:5.

9. Concentré d'agents nacrants, selon l'une des revendications 1 à 8, caractérisé en ce que l'alcool polyvalent de faible poids moléculaire comporte ? à 6 atomes de C et 2 à 6 groupes hydroxyle.

10. Concentré d'agents nacrants, selon la revendication 9, caractérisé en ce qu'il contient comme alcool polyvalent de faible poids moléculaire, du 1,2-propylèneglycol et/ou de la glycérine.

11. Procédé de préparation de concentrés d'agents nacrants, selon l'une des revendications 1 à 10, caractérisé en ce que l'on chauffe un mélange des composants (A), (B) et (c) à une température, située entre 1 et 30 °C au-delà du point de fusion, que l'on mélange avec la quantité d'eau nécessaire à environ la même température et que l'on refroidit ensuite à température ambiante.

12. Procédé de préparation de compositions aqueuses, liquides, troublées et nacrantes de substances tensioactives solubles dans l'eau, caractérisé en ce que l'on ajoute les concentrés d'agents nacrants selon l'une des revendications 1 à 10 aux préparations aqueuses claires, entre 0 et 40 °C, dans une proportion allant de 0,5 à 10 % en poids, en particulier de 1,5 à 5 % en poids de la préparation, que l'on répartit dans celle-ci sous agitation.